(19) Europäisches Patentamt
European Patent Office
Office européen des brevets

(11) **EP 4 279 600 A1**

(12) **EUROPEAN PATENT APPLICATION**
published in accordance with Art. 153(4) EPC

(43) Date of publication:
**22.11.2023 Bulletin 2023/47**

(21) Application number: **22752704.1**

(22) Date of filing: **07.02.2022**

(51) International Patent Classification (IPC):
**C12P 13/02** (2006.01)  **C12N 9/88** (2006.01)

(52) Cooperative Patent Classification (CPC):
**C12N 9/88; C12P 13/02**

(86) International application number:
**PCT/JP2022/004577**

(87) International publication number:
**WO 2022/172880 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.02.2021 JP 2021019409**

(71) Applicant: **Mitsubishi Chemical Corporation**
**Chiyoda-ku**
**Tokyo 100-8251 (JP)**

(72) Inventors:
• **YAMAGUCHI Takafumi**
**Tokyo 100-8251 (JP)**

• **HAGIYA Norifumi**
**Tokyo 100-8251 (JP)**
• **KANO Makoto**
**Tokyo 100-8251 (JP)**
• **TAKANASHI Kazuya**
**Tokyo 100-8251 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **IMPROVED NITRILE HYDRATASE REACTIVITY USING ALDEHYDE**

(57) The present invention relates to a method for producing an amide compound from a nitrile compound in the presence of a biocatalyst having nitrile hydratase activity, wherein the amide compound is efficiently produced by suppressing a deactivation of the biocatalyst, and improving the rate of conversion reaction from the nitrile compound to the amide compound.

EP 4 279 600 A1

**Description**

Technical Field

[Related Application]

The specification incorporates the content of the specification of Japanese Patent Application No. 2021-19409 (filed February 10, 2021) based on which the priority of this application is claimed.

[Technical field]

The present invention relates to a method for producing an amide compound by nitrile hydratase. More specifically, the present invention relates to a method for improving the reaction rate of nitrile hydratase, a method for suppressing deactivation of nitrile hydratase, and a method for producing an amide compound in the state where the reaction rate (of nitrile hydratase) is improved.

Background Art

Amide compounds have been widely used as substances industrially important. For example, acrylamide is used in a wide range of products including a coagulant for wastewater treatment, a paper strength enhancer and a petroleum recovery agent, whereas methacrylamide is widely used in products such as a paint and a glue.

As a method for producing an amide compound, an industrial method for producing an amide compound by hydrating the corresponding nitrile compound in the presence of conventionally reduced-state copper used as a catalyst, has been employed.

Recently, nitrile hydratase having nitrile hydration activity, that is, an activity to hydrate a nitrile group and convert it into an amide group, has been found in microbial enzymes, and a method for producing an amide compound from the corresponding nitrile compound using the enzyme or bacterial cells having the enzyme, has been used.

This production method is deemed to be an industrially excellent process over the conventional method using a metal catalyst, for the reasons that the reaction condition is mild; a nitrile compound is converted to the corresponding amide compound at a high conversion rate and with a high selectivity; with the result that a production process can be more simplified.

In producing an amide compound using nitrile hydratase industrially, it is important that an amide compound can be efficiently produced from the corresponding nitrile compound. It has been reported that the efficiency of producing an amide compound is enhanced by improving an enzymatic activity of nitrile hydratase (Patent Literature 1), suppressing a deactivation with temperature and improving the resistance of an amide compound (Patent Literature 2).

Other methods are reported in Patent Literatures 3 to 5, in which producibility is improved by identifying organic impurities affecting the activity of nitrile hydratase and contained in a nitrile compound, thereby suppressing deactivation of nitrile hydratase. For example, Patent Literature 3 reports a method of efficiently producing an amide compound by reducing the concentration of benzene present in a nitrile compound. A method of reducing the concentration of hydrocyanic acid present in a nitrile compound is reported in Patent Literatures 4 and 5.

Citation List

Patent Literatures

Patent Literature 1: Japanese Patent Laid-Open No. 2005-295815
Patent Literature 2: Japanese Patent Laid-Open No. 2010-172295
Patent Literature 3: International Publication No. WO2007-043466
Patent Literature 4: Japanese Patent Laid-Open No. H11-123098
Patent Literature 5: Japanese Patent Laid-Open No. 2001-288156

Summary of Invention

Technical Problem

The method of Patent Literature 4, however, has a problem. If a metal compound is added, it may affect an enzymatic reaction and the quality of the amide compound to be obtained. In addition, a step of removing the metal compound and the like is required after completion of the reaction, with the result that cost increases. In another case where a nitrile compound is purified with an ion exchange resin, the number of steps increases, with the result that cost increases.

In the method of Patent Literature 5, an alkali compound is added to a starting nitrile compound. An extra step for adjusting pH of a nitrile compound (solution) is required before an enzymatic reaction and the alkali compound may affect the quality of the resultant amide compound.

In the circumstances, a primary object of the present invention is to provide a method for improving productivity of an amide compound more simply by adding an aldehyde compound.

Solution to Problem

The present inventors conducted intensive studies in view of problems with the conventional technology. As a result, they found that addition of an aldehyde compound in a reaction for producing an amide compound from a nitrile compound using a biocatalyst having nitrile hydratase activity improves the rate of a conversion reaction from the nitrile compound to the amide compound and can suppress a deactivation of nitrile hydratase. Based on the finding, the present invention was accomplished.

More specifically, the present invention provides the following [1] to [10].

[1] A method for producing an amide compound from a nitrile compound in the presence of a biocatalyst having nitrile hydratase activity, wherein
a reaction that produces the amide compound from the nitrile compound is carried out in the presence of an aldehyde compound, and the nitrile compound is at least one selected from the group consisting of acrylonitrile, acetonitrile, methacrylonitrile, cyanopyridine, glycolonitrile and alanine nitrile.

[2] A method for improving a rate of a reaction that converts a nitrile compound to an amide compound in producing the amide compound from the nitrile compound in the presence of a biocatalyst having nitrile hydratase activity, comprising

a step of adding an aldehyde compound in a reaction solution containing the nitrile compound,
wherein the nitrile compound is at least one selected from the group consisting of acrylonitrile, acetonitrile, methacrylonitrile, cyanopyridine, glycolonitrile and alanine nitrile.

[3] A method for suppressing a deactivation of a biocatalyst having nitrile hydratase activity, in producing an amide compound from a nitrile compound in the presence of the biocatalyst having nitrile hydratase activity, comprising

a step of adding an aldehyde compound in a reaction solution containing the nitrile compound,
wherein the nitrile compound is at least one selected from the group consisting of acrylonitrile, acetonitrile, methacrylonitrile, cyanopyridine, glycolonitrile and alanine nitrile.

[4] The method according to any one of [1] to [3], wherein the ratio of the concentration of the aldehyde compound to the concentration of a cyan compound in a nitrile compound is from 0.9 to 15 by a molar ratio.
[5] The method according to any one of [1] to [5], wherein the biocatalyst having nitrile hydratase activity is nitrile hydratase derived from the genus *Rhodococcus* or *Pseudonocardia*; or cells, bacterial cells, or a processed material thereof containing the nitrile hydratase.
[6] The method according to any one of [1] to [4], wherein the aldehyde compound is at least one selected from the group consisting of formaldehyde, acetaldehyde, propionaldehyde, butyraldehyde, isobutyraldehyde, dialdehyde oxalate, malondialdehyde, pentanal, isovaleraldehyde, acrolein, crotonaldehyde, tiglic acid aldehyde, glyceraldehyde, glycolaldehyde, furfural, butanedial, trans-2-hexenal, glutaraldehyde, hexanal, heptanal, octanal, nonanal, decanal, paraldehyde, benzaldehyde, cinnamaldehyde, perillaldehyde, vanillin, 1-naphthoaldehyde, phthalaldehyde, methional, (Z)-7-hexadesenal, glyoxal (dialdehyde oxalate), paraformaldehyde, acetaldehyde ammonia and hexamethylenetetramine.
[7] A composition for producing an amide compound, comprising:

an aldehyde compound; and
at least one nitrile compound selected from the group consisting of acrylonitrile, acetonitrile, methacrylonitrile, cyanopyridine, glycolonitrile and lactonitrile.

[8] A catalyst composition for producing an amide compound, comprising an aldehyde compound and a biocatalyst having nitrile hydratase activity.

[9] An amide compound composition comprising an aldehyde compound and an amide compound containing a cyan compound, wherein the concentration of the cyan compound in the amide compound before the aldehyde compound is added, is 0.2 ppm or more.

[10] An amide compound composition comprising an amide compound, and a compound having a cyan compound and an aldehyde compound bound to each other.

Advantageous Effects of Invention

According to the present invention, it is possible to increase a rate of a reaction that converts a nitrile compound to an amide compound in the presence of a biocatalyst having nitrile hydratase activity by adding an aldehyde compound in a reaction solution containing a nitrile compound. According to the present invention, it is also possible to suppress deactivation of a biocatalyst having nitrile hydratase activity. In short, according to the present invention, an amide compound can be efficiently produced.

Description of Embodiments

Now, embodiments of the invention will be described.

(1) Biocatalyst Having Nitrile Hydratase Activity

In the present invention, nitrile hydratase refers to an enzyme having an ability to hydrolyze a nitrile compound to produce the corresponding amide compound. The biocatalyst having nitrile hydratase activity may be a protein of nitrile hydratase itself but an animal cell, a plant cell, an organelle or a bacterial cell containing nitrile hydratase and a processed material thereof may be used.

Examples of the processed material include a crushed material of an animal cell, a plant cell, an organelle or a bacterial cell; an enzyme extracted from a bacterial cell (crude enzyme or purified enzyme); and an animal cell, plant cell, organelle, bacterial cell or enzyme itself immobilized to a carrier.

Examples of the processed material also include an animal cell, a plant cell, an organelle or a bacterial cell that are chemically treated to lose proliferative ability.

Examples of an immobilization method include an enveloping method, a cross-linking method and a carrier binding method. The enveloping method is a coating method with a polymer membrane. The cross-linking method is a method of crosslinking enzyme with a reagent having two or more functional groups (polyfunctional cross-linking agent). The carrier binding method is a method of binding an enzyme to a water-insoluble carrier.

Examples of the carrier (immobilization carrier) for immobilization include glass beads, silica gel, polyurethane, polyacrylamide, polyvinyl alcohol, carrageenan, alginic acid, agar and gelatin.

Representative examples of the bacterial cell (microorganism) as mentioned above include microorganisms having nitrile hydratase activity and belonging to the genus *Rhodococcus, Gordona, Pseudomonas, Pseudonocardia, Geobacillus, Bacillus, Bacteridium, Micrococcus, Brevibacterium, Corynebacterium, Nocardia, Microbacterium, Fusarium, Agrobacterium, Acinetobacter, Xanthobacter, Streptomyces, Rhizobium, Klebsiella, Enterobacter, Erwinia, Pantoea, Candida, Aeromonas, Citrobacter* and *Achromobacter.*

More specifically, examples of the bacterial cell include *Nocardia* sp. N-775 disclosed in Japanese Patent Publication No. 56-17918; *Rhodococcus rhodochrous* J-1 disclosed in Japanese Patent Publication No. 06-55148; *Rhodococcus rhodochrous* NCIMB41164 strain disclosed in International Publication No. WO2005/054456: *Klebsiella* sp. MCI2609 disclosed in Japanese Patent Laid-Open No. H05-30982; *Aeromonas* sp. MCI2614 disclosed in Japanese Patent Laid-Open No. H05-30983; *Citrobacter freundii* MCI2615 disclosed in Japanese Patent Laid-Open No. H05-30984; *Agrobacterium rhizogenes* IAM13570 and *Agrobacterium faciens* disclosed in Japanese Patent Laid-Open No. H05-103681; *Xanthobacter flavas* JCM1204 and *Erwinia nigrifluens* MAFF03-01435 disclosed in Japanese Patent Laid-Open No. H05-161495; *Enterobacter* sp. MCI2707 disclosed in Japanese Patent Laid-Open No. H05-236975; *Streptomyces* sp. MCI2691 disclosed in Japanese Patent Laid-Open No. H05-236976; *Rhizobium* sp. MCI2610, *Rhizobium* sp. MCI2643, *Rhizobium* loti IAM13588, *Rhizobium leguminosarum,* IAM12609 and *Rhizobium merioti* IAM12611 disclosed in Japanese Patent Laid-Open No. H05-236977; *Candida guilliermondii* NH-2, *Pantoea agglomerans,* NH-3 and *Klebsiella pneumoniae* subsp. *pneumoniae* NH-26T2, disclosed in Japanese Patent Laid-Open No. H05-15384; *Agrobacterium radiobacter* SC-C15-1 disclosed in Japanese Patent Laid-Open No. H06-14786; *Bacillus smithii* SC-J05-1 disclosed in Japanese Patent Laid-Open No. H07-25494; *Pseudonocardia thermophila* ATCC19285 disclosed in Japanese Patent Laid-Open No. H08-56684; and *Pseudonocardia thermophila* JCM3095 disclosed in Japanese Patent Laid-Open No. H09-275978.

*Rhodococcus rhodochrous* J-1 strain disclosed in Japanese Patent Publication No. 06-55148 was deposited at the National Institute of Technology and Evaluation, Patent Biological Deposit Center (Central 6, 1-1-1 Higashi, Tsukuba, Ibaraki, Japan (hereinafter the same applies in the specification)) under accession number "FERM BP-1478", on September 18, 1987.

*Rhodococcus rhodochrous* NCIMB41164 strain disclosed in International Publication No. WO2005/054456 was deposited at the National Collection of Industrial, Food and Marine Bacteria, Ltd. (NCIMB) (NCIMB Ltd Ferguson Building Craibstone Estate Buksburn Aberdeen AB21 9YA) under accession number NCIMB41164 on March 5, 2003.

*Pseudonocardia thermophila* JCM3095 disclosed in Japanese Patent Laid-Open No. H09-275978 was deposited at the national institute of technology and evaluation patent biological deposit center (Central 6, 1-1-1 Higashi, Tsukuba, Ibaraki, Japan) under accession number "FERM BP-5785" on February 7, 1996.

In the present invention, one or two or more microorganisms selected from the aforementioned microorganisms can be used (alone or in combination).

A gene encoding nitrile hydratase can be introduced into a bacterial cell and expressed by a common molecular biological technique (as to the molecular technique, see Sambrook, Fritsch and Maniatis, "Molecular Cloning: A Laboratory Manual", 2nd Edition (1989), Cold Spring Harbor Laboratory Press). More specifically, in the present invention, it is possible to use an enzyme obtained by expressing a nucleic acid encoding natural nitrile hydratase (wild type) or a modified nitrile hydratase (mutant type) in a bacterial cell. In the present invention, one or two or more types of enzymes selected from the aforementioned enzymes can be used (alone or in combination).

The amino acid sequence of the wild-type nitrile hydratase is available from public database of NCBI such as GenBank (http: //www.ncbi.nlm.nih.gov/).

For example, the accession number of $\alpha$ subunit derived from *Rhodococcus rhodochrous* J1 (FERM BP-1478) is "P21219" and the accession number of $\beta$ subunit thereof is "P21220". The accession number of $\alpha$ subunit derived from *Rhodococcus rhodochrous* M8 (SU1731814) is "ATT79340" and the accession number of $\beta$ subunit thereof is "AAT79339". The accession number of $\alpha$ subunit derived from *Pseudomonas thermophila* JCM3095 is "1IRE A" and the accession number of $\beta$ subunit thereof is "1IREB".

Examples of a transformant into which a wild type nitrile hydratase gene is introduced include, but are not limited to, *Escherichia coli* MT10770 (FERM P-14756) (Japanese Patent Laid-Open No. H8-266277) transformed with nitrile hydratase (gene) of the genus *Achromobacter, Escherichia coli* MT10822 (FERM BP-5785) (Japanese Patent Laid-Open No. H9-275978) transformed with nitrile hydratase (gene) of the genus *Pseudonocardia,* or a microorganism transformed with nitrile hydratase (gene) (Japanese Patent Laid-Open No. H4-211379) derived from *Rhodococcus rhodochrous.*

Modified (mutant) nitrile hydratases obtained through amino acid substitution of a wild-type nitrile hydratase are known (e.g., Japanese Patent Laid-Open No. 2010-172295, Japanese Patent Laid-Open No. 2007-143409, Japanese Patent Laid-Open No. 2007-043910, Japanese Patent Laid-Open No. 2008-253182, Japanese Patent Laid-Open No. 2019-088326, Japanese Patent Laid-Open No. 2019-088327, WO05/116206, WO12/164933, WO12/169203, WO15/186298). In the method of the present invention, a microorganism having any one of (the genes) of these modified nitrile hydratases introduced therein can be used.

The microorganisms having nitrile hydratase activity mentioned above or processed materials thereof each can be used in a reaction for synthesizing an amide immediately after preparation of bacterial cells and stored after preparation of bacterial cells and used in a reaction for synthesizing an amide, as needed. A method for culturing a microorganism for preparing bacterial cells can be appropriately selected depending on the type of microorganism. In advance of main culture, seed culture may be carried out.

A bacterial cell having nitrile hydratase activity or a processed material thereof can be used for a batch reaction and a continuous reaction. As a reaction system, an appropriate system using, e.g., a fluidized bed, a fixed bed or a suspended bed may be selected. The temperature of a catalyst in a reaction solution herein is not particularly limited as long as it does not inhibit a mixing operation of an aqueous medium and a nitrile compound.

(2) Nitrile Compound

In the production method of the present invention, the nitrile compound to be used as a starting material is not particularly limited as long as it can be converted into an amide compound by a catalyst having nitrile hydratase activity. Examples of the nitrile compound include saturated aliphatic nitriles such as acetonitrile, propionitrile, succinonitrile, adiponitrile, glycolonitrile and lactonitrile; aliphatic unsaturated nitriles such as acrylonitrile and methacrylonitrile; aromatic nitriles such as benzonitrile and phthalodinitrile; and heterocyclic nitriles such as cyanopyridine. The nitrile compound of the present invention is preferably a nitrile compound such as acetonitrile, propionitrile, acrylonitrile, methacrylonitrile, n-butyronitrile, isobutyronitrile, cyanopyridine, glucolonitrile and lactonitrile; and particularly more preferably, acrylonitrile, methacrylonitrile, acetonitrile, cyanopyridine, glycolonitrile, and lactonitrile.

A nitrile compound becomes available as a commercial product generally through a purification step. For example, acrylonitrile is industrially produced by ammoxidation method of propylene; a cyan compound such as hydrocyanic acid is removed together with other byproducts by distillation operation (purification) performed after the reaction.

However, a cyan compound that cannot be completely removed by the distillation operation is contained in a product. A biocatalyst having nitrile hydratase activity may be damaged with the cyan compound contained in a nitrile compound, with the result that activity and reaction rate of the nitrile hydratase may reduce.

In the present invention, addition (presence) of an aldehyde compound in a reaction solution containing a nitrile compound can reduce the amount of a cyan compound contained in the nitrile compound and prevent the damage to a biocatalyst having nitrile hydratase activity by the cyan compound. In other words, the rate of conversion reaction from a nitrile compound to an amide compound carried out in the presence of a biocatalyst having nitrile hydratase activity is improved. Also, the deactivation of a biocatalyst having nitrile hydratase activity exposed to a nitrile compound can be conceivably suppressed.

(3) Production of Amide Compound

In the present invention, an amide compound is produced from a nitrile compound by using a biocatalyst having nitrile hydratase activity.

The type of amide compound to be produced in the present invention is not particularly limited and an amide compound can be produced in accordance with the use. As a starting material, a nitrile compound corresponding to a desired amide compound can be used.

Examples of the amide compound include acrylamide, nicotinamide and methacrylamide. Preferably, acrylamide is used.

If acrylonitrile is used as a nitrile compound, acrylamide is obtained. If methacrylonitrile is used as a nitrile compound, methacrylamide is obtained. If cyanopyridine is used as a nitrile compound, nicotinamide is obtained.

The method for producing an amide compound using a biocatalyst having nitrile hydratase activity is not particularly limited; for example, a continuous reaction process for continuously producing an amide compound or a batch reaction process for discontinuously producing an amide compound, may be employed.

In the case of the continuous reaction process, an amide compound can be continuously produced without taking out the whole reaction mixture from the reactor by continuously or discontinuously introducing a biocatalyst, water and a reactant containing a nitrile compound in a reactor, simultaneously with continuously or discontinuously taking out a reaction mixture containing a formed amide compound from the reactor.

In the case of the batch reaction process, an amide compound can be produced by supplying all reactants to a reactor at a time, and thereafter, carrying out a reaction, or supplying a portion of the reactants in a reactor, and continuously or intermittently supplying the remaining portion to the reactor, and then, carrying out a reaction.

Examples of the type of reactor include various types of reactors such as an agitation tank reactor, a fixed bed reactor, a fluidized bed reactor, a moving bed reactor, a tube reactor and a tower reactor. There reactors may be used singly or in combination. If a plurality of reactors are used, the more downstream the reactor is placed, the higher the concentration of an amide compound in the reaction mixture to be taken out. Because of this, the concentration of the amide compound finally obtained can be adjusted by the number of reactors.

When the reaction is continuously carried out by using a plurality of reactors, the reactor to which a biocatalyst having nitrile hydratase activity and a nitrile compound are to be introduced, is not limited only to the reactor positioned most upstream as long as, e.g., the reaction efficiency is not significantly declined; in other words, the biocatalyst and the nitrile compound can be introduced in the reactors positioned downstream of the most upstream reactor.

Of the reactants, water as a reactant is used for a hydration reaction of a nitrile compound for producing an amide compound.

Examples of the water as a reactant include water; or an aqueous solution containing an acid or a salt dissolved in water. Examples of the acid include phosphoric acid, acetic acid, citric acid and boric acid. Examples of the salt include a sodium salt, a potassium salt, an ammonium salt of any one of the acids mentioned above. Examples of the water as a reactant include, but are not limited to, pure water, city water, tris buffer, phosphate buffer, acetate buffer, citrate buffer and borate buffer. The pH (25°C) of the reactant water to be used is not particularly limited as long as a reaction in the presence of a biocatalyst can efficiently proceeds. For example, pH can be set at a value in the range of 4 to 10, and preferably 5 to 9. If pH is 4 or more, the enzymatic activity of a biocatalyst can be sufficiently enhanced. If pH is 10 or less, inactivation of a biocatalyst can be suppressed.

The amount of the biocatalyst to be used can be appropriately selected depending on, e.g., the type of biocatalyst to be used and the reaction condition. For example, the activity of the biocatalyst to be introduced in the reactor is preferably controlled so as to fall within the range of about 50 to 500 U per dry cell (1 mg) at a reaction temperature of 10°C. The unit "U" refers to the amount of the enzyme for producing one micromole of an amide compound from a nitrile compound per minute and is a value measured by using the nitrile compound to be used for production.

To the reactant for use in a hydration reaction of acrylonitrile or the reaction mixture during or after the hydration reaction, at least one type of water-soluble monocarboxylic acid salt having two or more carbon atoms may be added as a support measure for stabilization. The water-soluble monocarboxylic acid salt may be either a saturated monocarboxylic acid salt or an unsaturated monocarboxylic acid salt. Examples of the saturated carboxylic acid include acetic acid, propionic acid and n-caproic acid. Examples of the unsaturated carboxylic acid include acrylic acid, methacrylic acid and vinyl acetate. Representative examples of the salt include a sodium salt, a potassium salt and an ammonium salt. The addition amount of a water-soluble monocarboxylic acid salt is preferably 20 to 5000 mg/kg in terms of an acid relative to acrylamide in the reaction mixture (aqueous acrylamide solution) to be finally obtained.

The amount of the nitrile compound to be used can be appropriately selected depending on, e.g., the type of biocatalyst to be used and the reaction condition, reaction scale and continuous reaction or batch reaction.

The reaction temperature is not particularly limited as long as a biocatalyst can efficiently promote a reaction. The temperature is, for example, 5 to 50°C, preferably 10 to 40°C, and more preferably 15 to 35°C. If the reaction temperature is 10°C or more, the reaction activity of a biocatalyst can be sufficiently enhanced. If the reaction temperature is 50°C or less, the inactivation of a biocatalyst can be suppressed.

The reaction time is not particularly limited and can be appropriately selected depending on, for example, the reaction process such as batch reaction (batch reaction) and continuous reaction, and the reaction scale. The reaction time can be, for example, 0.1 to 60 hours, preferably 1 to 50 hours, and more preferably, 2 to 40 hours.

In the case where an amide compound is produced by a continuous reaction process, the fluid velocity in taking out a reaction mixture from a reactor may be determined in accordance with introduction rates of a nitrile compound and a biocatalyst such that a product can be continuously produced without taking out the whole amount of a reaction mixture in the reactor.

To stabilize a reaction, additives can be added to a nitrile compound or in a reaction solution.

The concentration of the amide compound in an aqueous solution of an amide compound obtained in the present invention, can be appropriately selected depending on, e.g., the intended use of the amide compound to be obtained. The concentration of an amide compound relative to the total mass of an aqueous solution of the amide compound to be obtained can be, for example, 25 to 65 mass%, preferably 30 to 60 mass%, and more preferably, 35 to 55 mass%. If the concentration of an amide compound is 65 mass% or less, it is possible to prevent precipitation of crystal of the amide compound at normal temperature. If the concentration of an amide compound is 25 mass% or more, it is possible to reduce the volume of a tank for storage or preservation and transportation cost.

The amide compound to be obtained by the present invention can be directly used in a polymerization reaction or stored until use. An aldehyde compound can be removed as needed. When an amide compound is stored, if necessary, the amide compound can be used by adding an additive including a polymerization inhibitor and a polymerization initiator.

(4) Aldehyde Compound

Herein, in the method for producing an amide compound from a nitrile compound in the presence of a biocatalyst having nitrile hydratase activity, a reaction from the nitrile compound to the amide compound is carried out in the presence of an aldehyde compound.

Herein, the aldehyde compound is not particularly limited as long as the aforementioned effect can be obtained. Examples of the aldehyde compound include formaldehyde, acetaldehyde, propionaldehyde, butyraldehyde, isobutyraldehyde, malondialdehyde, pentanal, isovaleraldehyde, acrolein, crotonaldehyde, tiglic acid aldehyde, glyceraldehyde, glycolaldehyde, furfural, butanedial, trans-2-hexenal, glutaraldehyde, hexanal, heptanal, octanal, nonanal, decanal, paraldehyde, benzaldehyde, cinnamaldehyde, perillaldehyde, vanillin, 1-naphthaldehyde, phthalaldehyde, methional and (Z)-7-hexadesenal.

Examples of aldehyde compounds include not only compounds having an aldehyde group (-CHO) but also compounds producing an aldehyde compound in water or in a solution. Examples of such aldehyde compounds include glyoxal (dialdehyde oxalate), paraformaldehyde, acetaldehyde ammonia and hexamethylenetetramine.

Of them, an aldehyde compound having a molecular weight of about 200 or less is preferable and an aldehyde compound having a molecular weight of about 100 or less is more preferable.

A particularly preferable aldehyde compound is, e.g., formaldehyde, acetaldehyde, propionaldehyde and butyraldehyde.

Note that, in the specification, the term "adding" to a reaction solution includes "allowing to be present" in a reaction solution.

In the present invention, the amount of an aldehyde compound to be added in a reaction solution containing a nitrile compound is not particularly limited as long as the rate of conversion reaction from a nitrile compound to an amide compound in the presence of a biocatalyst having nitrile hydratase activity can be increased or a deactivation of a biocatalyst having nitrile hydratase activity when the catalyst is exposed to a nitrile compound can be suppressed.

The amount (molar ratio) of an aldehyde compound to be added in a reaction solution relative to the content of a cyan compound in the reaction solution is set at 0.9 to 15, preferably 1.5 to 10.0, and more preferably 2.0 to 6.0.

The content of a cyan compound in acrylonitrile can be obtained by extracting it with an alkaline solution and subjecting the extract to titration with silver nitrate or can be measured by absorption photometry.

If the amount (molar ratio) of aldehyde, which is allowed to be present in a reaction solution relative to the content of a cyan compound is set at 0.9 or more, the rate of conversion reaction from a nitrile compound to an amide compound in the presence of a biocatalyst having nitrile hydratase activity can increase or a reduction of the activity of a biocatalyst having nitrile hydratase activity when the catalyst is exposed to a nitrile compound can be suppressed.

In contrast, the amount (molar ratio) of aldehyde, which is allowed to be present in a reaction solution relative to the content of a cyan compound is set at 15 or less. This is because even if the aldehyde compound is added in the amount (molar ratio) of more than 15, it is difficult to improve the (addition) effect.

Note that, in the specification, examples of the cyan compound include compounds containing cyan or releasing cyan or a cyan ion in a reaction condition, such as hydrocyanic acid (HCN), cyanide ion (CN⁻), sodium cyanide and potassium cyanide.

The timing at which an aldehyde compound is added in a reaction solution containing a nitrile compound is not particularly limited as long as the above effect can be produced. The timing may be before, at the time of (simultaneously with) and after exposure of a biocatalyst having nitrile hydratase activity to a nitrile compound (more specifically, after initiation of an enzymatic reaction of a nitrile compound by a biocatalyst).

Preferably, an aldehyde compound is added before a biocatalyst having nitrile hydratase activity is exposed to a nitrile compound. When an aldehyde compound is added after initiation of the enzymatic reaction, it should be added at a time not too long after initiation of the reaction (the sooner the aldehyde compound is added, the better), to obtain more effective results.

A method of adding an aldehyde compound in a reaction solution is not particularly limited as long as the aldehyde compound of a desired concentration is present in the reaction solution. An aldehyde compound can be added, for example, in a solid state or in a solution state where an aldehyde compound is dissolved in, e.g., a solvent or water, to be used for a reaction.

(5) Improvement of the Rate of Conversion Reaction from Nitrile Compound to Amide Compound

The present invention provides a method for improving a reaction rate of an enzymatic reaction that converts a nitrile compound to an amide compound (in producing a nitrile compound to an amide compound) in the presence of a biocatalyst having nitrile hydratase activity, characterized by including a step of adding an aldehyde compound in a reaction solution containing the nitrile compound. As the nitrile compound, at least one compound selected from the group consisting of acrylonitrile, acetonitrile, methacrylonitrile, cyanopyridine, glycolonitrile and alanine nitrile, can be used.

It has been reported that aldehyde is used for preventing (stabilizing) polymerization of acrylamide (WO2011/102510) but it was found for the first time in the present invention that an aldehyde compound prevents damage of a biocatalyst having nitrile hydratase activity and improves a reaction rate.

In the present invention, "improve a reaction rate" means that a reaction rate is high compared to the case where an aldehyde compound is not present.

Examples of a method for measuring a reaction rate include a method of measuring the amount of a nitrile compound present in the reaction system and reduced from the system per time, and a method of measuring the amount of an amide compound increased per time in a reaction system. As the measuring method for a nitrile compound or an amide compound, a method commonly known in the technical field, such as gas chromatographic analysis, can be used.

(6) Suppression of Deactivation of Biocatalyst Having Nitrile Hydratase Activity

There is provided a method for suppressing deactivation of a biocatalyst having nitrile hydratase activity characterized by including a step of adding an aldehyde compound in a reaction solution containing a nitrile compound in producing an amide compound from a nitrile compound in the presence of a biocatalyst having nitrile hydratase activity. As the nitrile compound, at least one compound selected from the group consisting of acrylonitrile, acetonitrile, methacrylonitrile, cyanopyridine, glycolonitrile and alanine nitrile, can be used.

As mentioned above, nitrile hydratase activity is susceptible to the effect of impurities in a reaction solution and reaction conditions. According to the method of the present invention, it is possible to easily suppress deactivation of nitrile hydratase without requiring an additional step or affecting the quality of an amide compound.

(7) Composition for Producing Amide Compound

The present invention provides a composition for producing an amide compound, comprising an aldehyde compound and at least one nitrile compound selected from the group consisting of acrylonitrile, acetonitrile, methacrylonitrile, cyanopyridine, glycolonitrile and lactonitrile.

In the composition for producing an amide compound of the present invention, an aldehyde compound specified in the section of "(4) Aldehyde Compound" can be used in accordance with the description in the section. The composition is used in the method for producing an amide compound specified in the section (3) and enables efficient production of an amide compound.

(8) Catalyst Composition for Producing Amide Compound

The present invention provides a catalyst composition for producing an amide compound, comprising an aldehyde compound and a biocatalyst having nitrile hydratase activity.

In the catalyst composition for producing an amide compound of the present invention, the aldehyde compound and the biocatalyst having nitrile hydratase activity are the same as specified in the section "(4) Aldehyde Compound" and the section "(1) Biocatalyst Having Nitrile Hydratase Activity", respectively.

(9) Amide Compound Composition

The present invention provides an amide compound composition containing an aldehyde compound and a cyan compound.

In a first embodiment, the amide compound composition contains an amide compound and an aldehyde compound, and is characterized in that the concentration of a cyan compound contained in the amide compound is 0.2 ppm or more. The cyan compound and amide compound may be separately present or bound to each other. The concentration of the cyan compound is defined as that before an aldehyde compound is added.

In a second embodiment, the amide compound composition contains an amide compound and a compound in which a cyan compound is bound to an aldehyde compound.

Examples

Now, the present invention will be more specifically described by way of Examples but the present invention is not limited to these Examples.

Note that, "%" in the specification represents "mass%".

[Example 1]

(Preparation of Bacterial Cells) *Rhodococcus rhodochrous* J-1 (FERMBP-1478)

*Rhodococcus rhodochrous* J-1 strain (FERMBP-1478) having nitrile hydratase activity was aerobically cultured in a medium (pH7.0) containing glucose (2.0%), urea (1.0%), peptone (0.5%), yeast extract (0.3%) and cobalt chloride (0.05%) at 30°C. The medium was washed with a 50 mM phosphate buffer (pH7.0) to obtain a bacterial suspension (3% on a dry-cell basis).

(Preparation of Acrylonitrile Containing Predetermined Concentration of Hydrocyanic Acid)

A cyan 1000-ppm standard solution (manufactured by Hayashi Pure Chemical Ind., Ltd.) was added to acrylonitrile for industrial use (manufactured by Mitsubishi Chemical Corporation) such that the hydrocyanic acid concentration became 2.1 ppm on a weight basis.

(Method for Measuring Hydrocyanic Acid Concentration)

To a test tube, 9.6 g of pure water and 0.4 g of acrylonitrile (manufactured by Mitsubishi Chemical Corporation) were added and the mixture was allowed to stand still at 25°C for 30 minutes. Thereafter, CyaniVer.3 contained in HACH analysis kit was added. The mixture was vortexed for 30 seconds and allowed to stand still for 30 seconds. CyaniVer.4 was added and the mixture was vortexed for 10 seconds. CyaniVer.5 was added and the mixture was vortexed for 2 minutes.

After the mixture was allowed to stand still at 25°C for 30 minutes, the absorbance of the mixture was measured by an absorption photometer (DR500001) (HACH company). As a result, the concentration of hydrocyanic acid was 2.1 ppm.

(Preparation of Acrylonitrile Containing Acetaldehyde)

To acrylonitrile for industrial use (manufactured by Mitsubishi Chemical Corporation), an acetaldehyde reagent (manufactured by FUJIFILM Wako Pure Chemical Corporation) was added so as to obtain 10 ppm on a weight basis.

(Reaction for Amide Formation)

To a glass case (internal volume: 13.5 ml) with a lid, 3.15 g of a phosphate buffer (pH7.0), and 3.15 g of a diluted bacterial cell solution, which was prepared by diluting bacterial cells as mentioned above with the phosphate buffer (pH7.0) such that initial activity thereof in a reaction solution became 1460 U, were added. The mixture was stirred while controlling the temperature at 20°C. To the mixture, acrylonitrile (4.8 mL) containing 10 ppm-acetaldehyde prepared in the above was added to initiate a reaction. Four hours later, the reaction solution was collected and the concentration of acrylonitrile was measured by gas chromatography (column: PoraPack-PS (manufactured by Waters Corporation), 1 m, 210°C, carrier gas: helium, detector: FID).

[Example 2]

The same procedure as in Example 1 was repeated except that the concentration of acetaldehyde in acrylonitrile was specified as 6.0 ppm.

[Example 3]

The same procedure as in Example 1 was repeated except that the concentration of acetaldehyde in acrylonitrile was specified as 3.0 ppm.

[Example 4]

The same procedure as in Example 1 was repeated except that the concentration of hydrocyanic acid in acrylonitrile was specified as 1.1 ppm.

[Comparative Example 1]

The same procedure as in Example 1 was repeated except that acetaldehyde was not added in acrylonitrile.

[Comparative Example 2]

The same procedure as in Example 1 was repeated except that the concentration of hydrocyanic acid in acrylonitrile was specified as 1.1 ppm and acetaldehyde was not added in acrylonitrile.

Table 1 shows the ratio of acrylonitrile concentration 4 hours later of initiation of the reaction relative to that of a case where an aldehyde compound was not added. The concentration ratio can be obtained in accordance with the following expression:

```
Concentration ratio [%] = (acrylonitrile concentration of

the case where aldehyde was added/the case where aldehyde

was not added) × 100
```

Note that, with respect to a compound forming an aldehyde compound in water or an aqueous solution, the ratio of aldehyde/cyan was calculated based on the molar number of the aldehyde compound formed in the water or aqueous solution.

[Table 1]

| | Concentration of hydrocyanic acid in acrylonitrile | Concentration of acetaldehyde in acrylonitrile | Acetaldehyde/ cyan [mol/mol] | Concentration ratio (4 hr) |
|---|---|---|---|---|
| | [ppm] | [ppm] | [-] | [%] |
| Example 1 | 2.1 | 10 | 2.9 | 38.8 |
| Example 2 | 2.1 | 6 | 1.8 | 76.1 |
| Example 3 | 2.1 | 3 | 0.9 | 92.5 |
| Example 4 | 1.1 | 10 | 5.6 | 9.8 |

In Examples where acetaldehyde was added, compared to Comparative Example where acetaldehyde was not added, even if the hydrocyanic acid concentration was 2.1 ppm or 1.1 ppm, the acrylonitrile concentration was low. From this, it was confirmed that the rate of a reaction that converts acrylonitrile to an amide compound is high.

[Test example 1]

The effects of the following compounds on the reaction for forming an amide compound were evaluated in accordance with Example 1.

No. 1: formaldehyde (30.03), No. 2: acetaldehyde (44.05), No. 3: propionaldehyde (58.08), No. 4: butyraldehyde (72.11), No. 5: hexanal (100.16), No. 6: heptanal (114.18), No. 7: octanal (128.21), No. 8: nonanal (142.24), No. 9: decanal (156.27), No. 10: formic acid (46.03), No. 11: dialdehyde oxalate (58.04), No. 12: meso-erythritol (122.12), No. 13: paraldehyde (132.16), No. 14: paraformaldehyde (30.03 × n), No. 15: benzaldehyde (106.12), No. 16: cinnamaldehyde (132.16), No. 17: perillaldehyde (150.22), No. 18: vanillin (152.15), No. 19: acetaldehyde ammonia (183.25), No. 20: hexamethylenetetramine (140.19).

The value within parentheses represents molecular weight (g/mol).

Compounds of No. 11, 14, 19, 20: aldehyde compound is formed in water.

Acrylonitrile was prepared so as to have a hydrocyanic acid concentration of 2.0 ppm.

To a glass case (internal volume: 13.5 ml) with a lid, 1.00 g of a diluted bacterial-cell solution, which was prepared by diluting bacterial cells as mentioned above with the phosphate buffer (pH7.0) such that initial activity thereof in a reaction solution became 1840 U, was added. A diluted solution containing an aldehyde compound diluted with a phosphate buffer (pH7.0) so as to obtain a concentration of 5 ppm or 10 ppm in the reaction solution, was added, and finally the phosphate buffer (pH7.0) was added to obtain a total amount of 6.2 g. The obtained solution was stirred while the temperature thereof was controlled at 25°C. To this solution, acrylonitrile (4.8 mL (acrylamide 50% equivalent, 38 wt%)) was added to initiate a reaction. At the time points of 3.5, 4.5, 5.0, and 6.0 hours later, the reaction solution was collected and the concentration of acrylonitrile was measured by gas chromatography (column: PoraPack-PS (manufactured by Waters Corporation), 1 m, 210°C, carrier gas: helium, detector: FID).

Table 2 shows the ratios of acrylonitrile concentration 3.5 hours and 6 hours after initiation of the reaction relative to that of the case where an aldehyde compound was not added. The concentration ratio can be obtained in accordance with the following expression:

```
Concentration ratio [%] = (acrylonitrile concentration of
the case where aldehyde was added/the case where aldehyde
was not added) × 100
```

Note that, with respect to a compound forming an aldehyde compound in water or an aqueous solution, the ratio of aldehyde/cyan was calculated based on the molar number of the aldehyde compound formed in the water or aqueous solution.

[Table 2]

| No. | Aldehyde, etc. | 5ppm Addition | | | Aldehyde/Cyan [mol/mol] [-] | 10ppm Addition | |
|---|---|---|---|---|---|---|---|
| | | Aldehyde/Cyan [mol/mol] [-] | Concentration Ratio (3.5 hr) [%] | Concentration Ratio (6 hr) [%] | | Concentration Ratio (3.5 hr) [%] | Concentration Ratio (6 hr) [%] |
| No. 1 | Formaldehyde | 5.8 | 0.1 | 1.8 | 11.6 | | |
| No. 2 | Acetaldehyde | 4.0 | 7.5 | 9.2 | 7.9 | | |
| No. 3 | Propionaldehyde | 3.0 | 2.2 | 1.4 | 6.0 | | |
| No. 4 | Butyraldehyde | 2.4 | 3.1 | 2.7 | 4.8 | | |
| No. 5 | Hexanal | 1.7 | 83.9 | 17.8 | 3.5 | | |
| No. 6 | Heptanal | 1.5 | 62.1 | 17.6 | 3.1 | | |
| No. 7 | Octanal | 1.4 | 106.4 | 117.5 | 2.7 | 72.9 | 11.6 |
| No. 8 | Nonanal | 1.2 | 97.1 | 81.4 | 2.5 | 80.1 | 35.0 |
| No. 9 | Decanal | 1.1 | 96.1 | 67.0 | 2.2 | | |
| No. 10 | Formic Acid | 3.8 | 99.6 | 88.9 | 7.6 | 95.1 | 94.9 |
| No. 11 | Dialdehyde Oxalate | 3.0 | 33.4 | 12.2 | 6.0 | | |
| No. 12 | meso-Erythritol | 1.4 | 103.3 | 118.2 | 2.9 | 97.8 | 97.3 |
| No. 13 | Paraldehyde | 1.3 | | | 2.6 | | |
| No. 14 | Paraformaldehyde | 5.8 | 1.1 | 1.1 | 11.6 | - | |

(continued)

| No. | Aldehyde, etc. | 5ppm Addition | | | 10ppm Addition | | |
|-----|----------------|----------------------------|------------------------------------|--------------------------------|----------------------------|------------------------------------|--------------------------------|
|     |                | Aldehyde/Cyan [mol/mol] [-] | Concentration Ratio (3.5 hr) [%] | Concentration Ratio (6 hr) [%] | Aldehyde/Cyan [mol/mol] [-] | Concentration Ratio (3.5 hr) [%] | Concentration Ratio (6 hr) [%] |
| No. 15 | Benzaldehyde | 1.7 | 92.0 | 66.2 | 3.3 | | |
| No. 16 | Cinnamaldehyde | 1.3 | 97.0 | 102.6 | 2.6 | 99.2 | 87.1 |
| No. 17 | Perillaldehyde | 1.2 | 82.8 | 59.6 | 2.3 | | - |
| No. 18 | Vanillin | 1.2 | 92.9 | 71.7 | 2.3 | - | |
| No. 19 | Acetaldehyde Ammonia | 1.0 | 30.0 | 3.3 | 1.9 | - | |
| No. 20 | Hexamethylenetetramine | 7.5 | 0.3 | 2.4 | 15.0 | - | |

With respect to aldehyde compounds except formic acid, meso-erythritol and paraldehyde, it was confirmed that the acrylonitrile concentration was reduced and the reaction rate of converting a nitrile compound to an amide compound was improved in the case where an aldehyde compound was added compared to the case where an aldehyde compound was not added.

[Example 5]

(Preparation of Transformant Having Nitrile Hydratase derived from *Rhodococcus Rhodochrous* M8 Strain)

(1) Preparation of Chromosomal DNA From *Rhodococcus Rhodochrous* M8 Strain (hereinafter referred to as M8 strain)

M8 strain (SU1731814) is available from the Russian Strain Center IBFM (VKPM S-926) .

M8 strain was subjected to shaking culture performed in a medium (pH7.0) of 100 mL MYK (0.5% polypeptone, 0.3% Bacto yeast extract, 0.3% Bacto malt extract, 0.2% $K_2HPO_4$, 0.2% $K_2HPO_4$) at 30°C for 72 hours. The culture solution was centrifuged. The bacterial cells collected were suspended in 4 mL of a Saline-EDTA solution (0.1 M EDTA, 0.15 M NaCl (pH8.0)). To the suspension, lysozyme (8 mg) was added. After shaken at 37°C for one to two hours, the suspension was frozen at -20°C.

Subsequently, to the suspension, 10 mL of Tris-SDS solution (1% SDS, 0.1 M NaCl, 0.1 M Tris-HCl (pH9.0)) was gently added while shaking. Further, to the suspension, proteinase K (manufactured by Merck) (final concentration 0.1 mg) was added and the mixture was shaken at 37°C for one hour. Subsequently, an equal amount of TE-saturated phenol was added. After the mixture was stirred (TE: 10 mM Tris-HCl, 1 mM EDTA (pH8.0)), it was centrifuged. The upper layer was collected and a double volume of ethanol was added. DNA was rolled up (taken up) by a glass rod. Thereafter, DNA was centrifuged successively with 90%, 80% and 70% ethanol to remove phenol.

Subsequently, DNA was dissolved with 3 mL of a TE buffer solution and ribonuclease A solution (treated with heat at 100°C for 15 minutes) was added thereto so as to obtain a concentration of 10 μg/mL. The mixture was shaken at 37°C for 30 minutes. Further, proteinase K (manufactured by Merck) was added and the mixture was shaken at 37°C for 30 minutes. To this mixture, an equal volume of TE-saturated phenol was added. After the mixture was centrifuged, the upper layer and the lower layer were separated.

To the upper layer, further an equal volume of TE-saturated phenol was added. After the mixture was centrifuged, the upper layer and the lower layer were separated. This operation was repeated again. Thereafter, to the upper layer, the same volume of chloroform (containing 4% isoamyl alcohol) was added. After the mixture was centrifuged, the upper layer was recovered. Then, to the upper layer, a double volume of ethanol was added and DNA was recovered by taking it up by a glass rod to obtain chromosomal DNA.

(2) Preparation of Plasmid Expressing Nitrile Hydratase derived from M8 Strain

The nitrile hydratase gene (SEQ ID NO:5) derived from M8 strain is disclosed in a Non Patent Literature (Veiko, V.P. et al, Cloning, nucleotide sequence of nitrile hydratase gene from Rhodococcus rhodochrous M8, Biotekhnologiia (Mosc.), 5, 3-5 (1995)). The amino acid sequences of β subunit, α subunit and activator are represented by SEQ ID NO:6, SEQ ID NO:7 and SEQ ID NO:8, respectively. Using the following primers (SEQ ID NO:1, 2), which were synthesized based on these sequence information, and M8 strain genomic DNA prepared as a template, PCR was carried out in the following reaction conditions.

Primers:

M8-1: 5'-GGTCTAGAATGGATGGTATCCACGACACAGGC-3' (SEQ ID NO: 1)
M8-2: 5'-cccctgcaggtcagtcgatgatggccatcgattc-3' (SEQ ID NO:2)

Composition of reaction solution:

| | |
|---|---|
| Template DNA (M8 strain genome DNA) | 1 μl |
| Primer M8-1 (SEQ ID NO:15) | 0.5 μl |
| Primer M8-2 (SEQ ID NO:16) | 0.5 μl |

(continued)

| | |
|---|---|
| Sterilized Water | 8 μl |
| PrimeSTAR (Takara Bio Inc.) | 10 μl |
| Total Amount | 20 μl |

Temperature cycle:

A cycle consisting of a reaction of 98°C for 10 seconds, a reaction of 55°C for 5 seconds and a reaction of 72°C for 30 seconds was repeated 30 times.

The obtained plasmid DNA was digested with restriction enzymes XbaI and Sse8387I and subjected to electrophoresis by 0.7% agarose gel. A nitrile hydratase gene fragment (SEQ ID NO:5) of 1.6 kb was recovered and introduced into the XbaI-Sse8387I site of plasmid pSJ042. The obtained plasmid was designated as pSJ-N01A. Note that, pSJ042 was prepared as a plasmid expressing J1 strain-derived nitrile hydratase in *Rhodococcus* in accordance with a method disclosed in Japanese Patent Laid-Open No. 2008-154552. Plasmid pSJ023 used for preparation of pSJ042 was deposited as a transformant ATCC12674/pSJ023 (FERM BP-6232) at the National Institute of Advanced Industrial Science and Technology, Patent Biological Deposit Center (Central 6, 1-1-1 Higashi, Tsukuba, Ibaraki, Japan) on March 4, 1997.

(3) Preparation of ATCC12674 Transformant

Bacterial cells of *Rhodococcus rhodochrous* ATCC 12674 strain in the logarithmic growth phase were centrifugally collected, washed three times with sterilized water cooled on ice, and suspended with sterilized water to prepare competent cells.

Plasmid DNA (pSJ-N01A) (1 μl) prepared in the above and ATCC12674 competent cells (10 μl) were mixed and cooled on ice for 30 minutes. The suspension of the DNA and the bacterial cells was put in cuvettes and irradiated with electric pulse by Gene Pulser (BIO RAD) at 20 kV/cm, 200 OHMS. The suspension irradiated with electric pulse was allowed to stand still under ice cooling for 10 minutes and subjected to heat-shock performed at 37°C for 10 minutes. To the resultant suspension, 500 μl of MYK medium (0.5% polypepton, 0.3% Bacto yeast extract, 0.3% Bacto malt extract, 0.2% $K_2HPO_4$, 0.2% $K_2HPO_4$) was added. The mixture was allowed to stand still at 30°C for 24 hours, smeared on a 10 μg/ml kanamycin-containing MYK agar medium and cultured at 30°C for three days.

The plasmid in colonies was checked to obtain a transformant (ATCC12674/pSJ-N01A).

(4) Culture of Recombinant Bacterium

The transformant (ATCC12674/pSJ-N01A) obtained in the above step was inoculated in MYK medium (50 μg/ml kanamycin) and subjected to shaking culture performed at 30°C for two days. The cultured transformant (1%) was inoculated in GGPK medium (1.5% glucose, 1% monosodium glutamate, 0.1% yeast extract, 0.05% $K_2HPO_4$, 0.05% $KH_2PO_4$, 0.05% $Mg_2O_4 \cdot 7H_2O$, 1% $CoCl_2$, 0.1% urea, 50 μg/ml kanamycin, pH7.2). Shaking culture was carried out at 30°C for three days and bacterial cells were centrifugally collected. Thereafter, the bacterial cells were washed with a 100 mM phosphate buffer (pH7.0) and a bacterial suspension was prepared.

(5) Evaluation of Effect on Amide Compound Formation Reaction

According to Example 1, the effect of propionaldehyde on amide compound formation reaction by M8 strain-derived transformant (ATCC12674/pSJ-N01A) was evaluated.

Acrylonitrile was prepared so as to have a hydrocyanic acid concentration of 2.0 ppm.

To a glass case (internal volume: 13.5 ml) with a lid, 1.00 g of a diluted bacterial-cell solution, which was prepared by diluting bacterial cells as mentioned above with the phosphate buffer (pH7.0) such that the initial amount of the bacterial cells in a reaction solution became 5.6 mg; and a diluted solution, which was prepared by diluting each aldehyde compound with a phosphate buffer (pH7.0) so as to obtain a concentration thereof of 10 ppm, 25 ppm or 50 ppm in a reaction solution, were added; and finally, a phosphate buffer (pH7.0) was added up to a total amount of 9.0 g. The solution obtained was stirred while the temperature thereof was controlled at 25°C. To this solution, acrylonitrile (1.0 g) was added to initiate a reaction. Six hours later, the reaction solution was collected and the concentration of acrylonitrile was measured by gas chromatography (column: PoraPack-PS (manufactured by Waters Corporation), 1 m, 210°C, carrier gas: helium, detector: FID).

Table 3 shows the ratio of acrylonitrile concentration 6 hours after initiation of the reaction relative to that of a case where an aldehyde compound was not added. The concentration ratio can be obtained in accordance with the

following expression:

$$\text{Concentration ratio [\%]} = (\text{acrylonitrile concentration of}$$
$$\text{the case where aldehyde was added/the case where aldehyde}$$
$$\text{was not added}) \times 100$$

Note that, with respect to a compound forming an aldehyde compound in water or an aqueous solution, the ratio of aldehyde/cyan was calculated based on the molar number of the aldehyde compound formed in the water or aqueous solution.

[Table 3]

| Concentration of propionaldehyde in a reaction solution [ppm] | Propionaldehyde/cyan [mol/mol] [-] | Concentration ratio (6 hr) [%] |
|---|---|---|
| 10 | 22.9 | 82.7 |
| 25 | 57.1 | 70.4 |
| 50 | 114.3 | 60.0 |

In M8 strain-derived transformant (ATCC12674/pSJ-N01A), the acrylonitrile concentration is low in the case where an aldehyde compound was added compared to the case where an aldehyde compound was not added. From this, it was confirmed that the reaction rate of converting a nitrile compound to an amide compound is improved by addition of an aldehyde compound.

[Example 6]

(1) Preparation of DN1 Transformant Expressing Nitrile Hydratase Derived from *Pseudonocardia Thermophila* JCM3095 Strain

Plasmid pPT-DB1 is a plasmid containing a nitrile hydratase gene derived from *Pseudonocardia thermophila* JCM3095 strain (hereinafter referred to as JCM3095 strain) obtained in Japanese Patent Laid-Open No. H9-275978 and has been deposited, as a transformant (MT-10822 strain) introduced in *Escherichia coli* HB101 at the National Institute of Advanced Industrial Science and Technology (Central 6, 1-1-1 Higashi, Tsukuba, Ibaraki, Japan).

The nitrile hydratase gene (SEQ ID NO:9) derived from JCM3095 strain is disclosed in Japanese Patent Laid-Open No. H9-275978. The amino acid sequences of β subunit, α subunit and activator are represented by SEQ ID NO:10, SEQ ID NO:11 and SEQ ID NO:12, respectively. Using the following primers (SEQ ID NO:3, 4) synthesized based on these sequence information, and pPT-DB1 as a template, PCR was carried out in the following reaction conditions. pRT-DB1 used as a template was prepared from MT-10822 strain in accordance with a routine method.

Primers:

PSN-1: 5'-GGTCTAGAATGAACGGCGTGTACGACGTCGGC-3' (SEQ ID NO:3)
PSN-2: 5'-ccCCTGCAGGTCAGGACCGCACGGCCGGGTGGAC-3' (SEQ ID NO:4)

Composition of reaction solution:

| | |
|---|---|
| Template DNA (pPT-DB1) | 1 μl |
| Primer PSN-1 (SEQ ID NO:17) | 0.5 μl |
| Primer PSN-2 (SEQ ID NO:18) | 0.5 μl |
| Sterilized Water | 8 μl |

(continued)

| | |
|---|---|
| PrimeSTAR (Takara Bio Inc.) | 10 μl |
| Total Amount | 20 μl |

Temperature cycle:

A cycle consisting of a reaction at 98°C for 10 seconds, a reaction at 55°C for 5 seconds and a reaction at 72°C for 30 seconds was repeated 30 times.

A plasmid was prepared by using the PCR product (SEQ ID NO:10) obtained in the same manner as in Example 5 (2) and designated as pSJ-N02A. The plasmid obtained was introduced into ATCC12674 in the same manner as in Example 5 (3) to obtain a transformant (ATCC12674/pSJ-N02A).

(4) Culture of Recombinant Bacterium

The transformant (ATCC12674/pSJ-N02A) obtained in the above step was inoculated in MYK medium (50 μg/ml kanamycin) and subjected to shaking culture performed at 30°C for two days. The cultured transformant (1%) was inoculated in GGPK medium (1.5% glucose, 1% monosodium glutamate, 0.1% yeast extract, 0.05% $K_2HPO_4$, 0.05% $KH_2PO_4$, 0.05% $Mg_2O_4 \cdot 7H_2O$, 1% $CoCl_2$, 0.1% urea, 50 μg/ml kanamycin, pH7.2). Shaking culture was carried out at 30°C for three days and bacterial cells were centrifugally collected. Thereafter, the bacterial cells were washed with a 100 mM phosphate buffer (pH7.0) and a bacterial suspension was prepared.

(5) Evaluation of Effect on Amide Compound Formation Reaction

According to Example 1, the effect of propionaldehyde on amide compound formation reaction by transformant (ATCC12674/pSJ-N02A) was evaluated.

Acrylonitrile was prepared so as to have a hydrocyanic acid concentration of 2.0 ppm.

To a glass case (internal volume: 13.5 ml) with a lid, 1.00 g of a diluted bacterial-cell solution, which was prepared by diluting the bacterial cells as mentioned above with a phosphate buffer (pH7.0) such that the initial amount of the bacterial cells in a reaction solution became 4.1 mg, was added; and a diluted solution, which was prepared by diluting each aldehyde compound with a phosphate buffer (pH7.0) so as to obtain a concentration thereof of 7 ppm, in a reaction solution, was added; and finally, a phosphate buffer (pH7.0) was added up to a total amount of 8.5 g. The solution obtained was stirred while the temperature thereof was controlled at 25°C. To this solution, acrylonitrile (1.5 g) was added to initiate a reaction. Six hours later, the reaction solution was collected and the concentration of acrylonitrile was measured by gas chromatography (column: PoraPack-PS (manufactured by Waters Corporation), 1 m, 210°C, carrier gas: helium, detector: FID) .

Table 4 shows the ratio of acrylonitrile concentration 6 hours after initiation of the reaction relative to that of the case where an aldehyde compound was not added. The concentration ratio can be obtained in accordance with the following expression:

```
Concentration ratio [%] = (acrylonitrile concentration of

the case where aldehyde was added/the case where aldehyde

was not added) × 100
```

Note that, with respect to a compound forming an aldehyde compound in water or an aqueous solution, the ratio of aldehyde/cyan was calculated based on the molar number of the aldehyde compound formed in the water or aqueous solution.

[Table 4]

| Concentration of propionaldehyde in a reaction solution [ppm] | Propionaldehyde/cyan [mol/mol] [-] | Concentration ratio (6 hr) [%] |
|---|---|---|
| 7 | 10. 7 | 3 3. 3 |

The acrylonitrile concentration is low in the case where an aldehyde compound was added compared to the case where an aldehyde compound was not added. From this, it was confirmed that the reaction rate of converting a nitrile compound to an amide compound is improved by addition of an aldehyde compound.

Industrial Applicability

The present invention is useful in biological industrial production of an amide compound such as acrylamide and methacrylamide from a nitrile compound.

All publications, patents and patent applications cited in the specification are incorporated by reference herein in their entireties.

[Sequence Listing Free Text]

> SEQ ID NO:1: primer M8-1
> SEQ ID NO:2: primer M8-2
> SEQ ID NO:3: primer PSN-1
> SEQ ID NO:4: primer PSN-2

**Claims**

1. A method for producing an amide compound from a nitrile compound in the presence of a biocatalyst having nitrile hydratase activity, wherein
a reaction that produces the amide compound from the nitrile compound is carried out in the presence of an aldehyde compound, and the nitrile compound is at least one selected from the group consisting of acrylonitrile, acetonitrile, methacrylonitrile, cyanopyridine, glycolonitrile and alanine nitrile.

2. A method for improving a rate of a reaction that converts a nitrile compound to an amide compound in producing the amide compound from the nitrile compound in the presence of a biocatalyst having nitrile hydratase activity, comprising

   > a step of adding an aldehyde compound in a reaction solution containing the nitrile compound,
   > wherein the nitrile compound is at least one selected from the group consisting of acrylonitrile, acetonitrile, methacrylonitrile, cyanopyridine, glycolonitrile and alanine nitrile.

3. A method for suppressing a deactivation of a biocatalyst having nitrile hydratase activity, in producing an amide compound from a nitrile compound in the presence of the biocatalyst having nitrile hydratase activity, comprising

   > a step of adding an aldehyde compound in a reaction solution containing the nitrile compound,
   > wherein the nitrile compound is at least one selected from the group consisting of acrylonitrile, acetonitrile, methacrylonitrile, cyanopyridine, glycolonitrile and alanine nitrile.

4. The method according to any one of claims 1 to 3, wherein the ratio of the concentration of the aldehyde compound to the concentration of a cyan compound in a nitrile compound is from 0.9 to 15 by a molar ratio.

5. The method according to any one of claims 1 to 4, wherein the biocatalyst having nitrile hydratase activity is nitrile hydratase derived from the genus *Rhodococcus* or *Pseudonocardia;* or cells, bacterial cells, or a processed material thereof containing the nitrile hydratase.

6. The method according to any one of claims 1 to 4, wherein the aldehyde compound is at least one selected from the group consisting of formaldehyde, acetaldehyde, propionaldehyde, butyraldehyde, isobutyraldehyde, dialdehyde oxalate, malondialdehyde, pentanal, isovaleraldehyde, acrolein, crotonaldehyde, tiglic acid aldehyde, glyceraldehyde, glycolaldehyde, furfural, butanedial, trans-2-hexenal, glutaraldehyde, hexanal, heptanal, octanal, nonanal, decanal, paraldehyde, benzaldehyde, cinnamaldehyde, perillaldehyde, vanillin, 1-naphthoaldehyde, phthalaldehyde, methional, (Z)-7-hexadesenal, glyoxal (dialdehyde oxalate), paraformaldehyde, acetaldehyde ammonia and hexamethylenetetramine.

7.  A composition for producing an amide compound, comprising:

    an aldehyde compound; and
    at least one nitrile compound selected from the group consisting of acrylonitrile, acetonitrile, methacrylonitrile, cyanopyridine, glycolonitrile and lactonitrile.

8.  A catalyst composition for producing an amide compound, comprising an aldehyde compound and a biocatalyst having nitrile hydratase activity.

9.  An amide compound composition comprising an aldehyde compound and an amide compound containing a cyan compound, wherein the concentration of the cyan compound in the amide compound before the aldehyde compound is added, is 0.2 ppm or more.

10. An amide compound composition comprising an amide compound, and a compound having a cyan compound and an aldehyde compound bound to each other.

## INTERNATIONAL SEARCH REPORT

| International application No. |
| --- |
| **PCT/JP2022/004577** |

### A. CLASSIFICATION OF SUBJECT MATTER

*C12P 13/02*(2006.01)i; *C12N 9/88*(2006.01)i
FI:   C12P13/02 ZNA; C12N9/88

According to International Patent Classification (IPC) or to both national classification and IPC

### B. FIELDS SEARCHED

Minimum documentation searched (classification system followed by classification symbols)

C12P13/02; C12N9/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2017-184686 A (MITSUBISHI CHEM HOLDINGS CORP) 12 October 2017 (2017-10-12) claims, paragraphs [0014], [0052], examples 1, 8 | 1-8 |
| Y | | 9 |
| X | JP 2010-22334 A (MITSUBISHI RAYON CO LTD) 04 February 2010 (2010-02-04) paragraphs [0011]-[0013], [0107]-[0109] | 10 |
| X | JP 2002-369697 A (DAICEL CHEM IND LTD) 24 December 2002 (2002-12-24) paragraph [0068] | 10 |
| Y | JP 6020741 B1 (MITSUBISHI RAYON CO LTD) 02 November 2016 (2016-11-02) paragraph [0031] | 9 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 April 2022** | **12 April 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/004577**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/004577**

| Patent document cited in search report | | | Publication date (day/month/year) | Patent family member(s) | | | Publication date (day/month/year) |
|---|---|---|---|---|---|---|---|
| JP | 2017-184686 | A | 12 October 2017 | (Family: none) | | | |
| JP | 2010-22334 | A | 04 February 2010 | (Family: none) | | | |
| JP | 2002-369697 | A | 24 December 2002 | US paragraph [0101] EP | 2003/0124691 1266962 | A1 A2 | |
| JP | 6020741 | B1 | 02 November 2016 | CN paragraph [0056] WO | 106795227 2016/098269 | A A1 | |

Form PCT/ISA/210 (patent family annex) (January 2015)

22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021019409 A
- JP 2005295815 A
- JP 2010172295 A
- WO 2007043466 A
- JP H11123098 A
- JP 2001288156 A
- JP 56017918 A
- JP 6055148 A
- WO 2005054456 A
- JP H0530982 A
- JP H0530983 A
- JP H0530984 A
- JP H05103681 A
- JP H05161495 A
- JP H05236975 A
- JP H05236976 A
- JP H05236977 A
- JP H0515384 A
- JP H0614786 A
- JP H0725494 A
- JP H0856684 A
- JP H09275978 A
- JP H8266277 A
- JP H9275978 A
- JP H4211379 A
- JP 2007143409 A
- JP 2007043910 A
- JP 2008253182 A
- JP 2019088326 A
- JP 2019088327 A
- WO 05116206 A
- WO 12164933 A
- WO 12169203 A
- WO 15186298 A
- WO 2011102510 A
- SU 1731814
- JP 2008154552 A

**Non-patent literature cited in the description**

- **SAMBROOK ; FRITSCH ; MANIATIS.** Molecular Cloning: A Laboratory Manual. Cold Spring Harbor Laboratory Press, 1989
- **VEIKO, V.P. et al.** Cloning, nucleotide sequence of nitrile hydratase gene from Rhodococcus rhodochrous M8. *Biotekhnologiia,* 1995, vol. 5, 3-5